(19) Europäisches Patentamt
European Patent Office
Office européen des brevets

(11) Publication number: **0 073 845**
**B1**

# (12) EUROPEAN PATENT SPECIFICATION

(45) Date of publication of patent specification: **29.05.85**

(51) Int. Cl.[4]: **C 07 D 471/20,** A 61 K 31/55, A 61 K 31/445

(21) Application number: **81106872.5**

(22) Date of filing: **03.09.81**

## (54) Imidazopyridine-spiro-piperidine compounds.

(43) Date of publication of application:
**16.03.83 Bulletin 83/11**

(45) Publication of the grant of the patent:
**29.05.85 Bulletin 85/22**

(84) Designated Contracting States:
**BE DE FR GB SE**

(56) References cited:
**WO-A-81/01554**
**US-A-3 123 610**
**US-A-3 329 683**
**US-A-3 637 713**
**US-A-3 842 076**

**PATENT ABSTRACTS OF JAPAN, unexamined application section C, vol. 2, no. 93, July 29, 1978, THE PATENT OFFICE JAPANESE GOVERNMENT, page 1518 C 78**
**E. SCHRÖDER et al., Arzneimittelchemie I, Georg Thieme Verlag, Stuttgart, Seite 292 (1976**

**The file contains technical information submitted after the application was filed and not included in this specification**

(73) Proprietor: **Yoshitomi Pharmaceutical Industries, Ltd.**
**35 Hiranomachi 3-chome Higashi-ku**
**Osaka-shi Osaka 541 (JP)**

(72) Inventor: **Tashiro, Chiaki**
**792-1 Oaza-Hirotsu Yoshitomimachi**
**Chikujogun Fukuoka 871 (JP)**
Inventor: **Horii, Ichiro**
**1336 Oaza-Hirotsu Yoshitomimachi**
**Chikujogun Fukuoka 871 (JP)**

(74) Representative: **von Kreisler, Alek, Dipl.-Chem. et al**
**Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 073 845 B1**

## Description

This invention relates to novel imidazopyridine-spiro-piperidine compounds which are therapeutically useful as psychotropics.

US—A—3 842 076 and E. Schröder et al Arzneimittelchemie I, Georg Thieme Verlag, Stuttgart, p. 292 (1976) are considered the closest prior art.

According to the present invention, there is provided an imidazopyridine-spiro-piperidine compound of the formula:

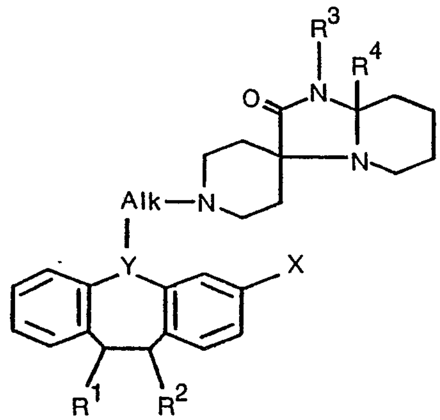

(I)

or a pharmaceutically acceptable acid addition salt thereof, wherein X represents hydrogen, halogen (e.g. F, Cl or Br) or lower alkoxy selected from methoxy, ethoxy, propoxy or butoxy, Y represents

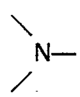

or

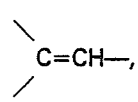

$R^1$ and $R^2$ both represent hydrogen or combinedly form a single bond, Alk represents lower alkylene selected from ethylene, propylene or trimethylene, and $R^3$ and $R^4$ both represent hydrogen or combinedly form a single bond.

The compounds of formula (I) can be produced by reacting a compound of the formula:

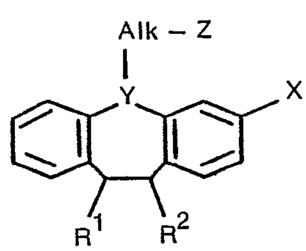

wherein X, Y, $R^1$, $R^2$ and Alk are as defined above, and Z represents a reactive atom or group (e.g. Cl, Br, methylsulfonyloxy or p-tolylsulfonyloxy), with a compound of the formula:

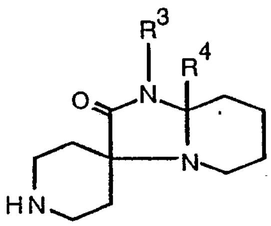

(III)

wherein $R^3$ and $R^4$ are as defined above.

The reaction is usually carried out in an inert solvent (preferably ethanol), if necessary in the presence of an acid acceptor (e.g. sodium amide, sodium hydroxide, sodium carbonate or potassium carbonate), at a temperature of from room temperature to the boiling point of the solvent employed, for a period of several hours to several tens of hours.

The novel starting compounds of formula (III) can be prepared, for example, by heating 1 - benzyl - 4 - carbamoyl - 4 - piperidino - piperidine or 4 - carbamoyl - 4 - piperidino - piperidine in an aqueous medium in the presence of a catalyst for catalytic reduction (preferably palladium-on-charcoal catalyst).

*Preparation of Starting Compounds*

Preparative Example A

A mixture of 10 g of 1-benzyl-4-carbamoyl-4-piperidino-piperidine, 3 g of 5% palladium-on-charcoal catalyst and 100 ml of water is boiled under reflux for 8 hours, while the toluene formed is removed. The reaction mixture is then cooled, and the catalyst is removed by filtration. The filtrate is concentrated to 10 ml under reduced pressure and is allowed to stand. The precipitated crystals are collected by filtration, dried and recrystallized from chloroform to give 2,3,5,6,7,8 - hexahydro - 2 - oxo - imidazo[1,2 - a]pyridine - 3 - spiro - 4′ - piperidine as colorless crystals, m.p. 220°C, 70% yield.

Preparative Example B

A mixture of 5 g of 4-carbamoyl-4-piperidino-piperidine, 0.5 g of 5% palladium-on-charcoal catalyst and 50 ml of water is boiled under reflux for 50 hours. The reaction mixture is then cooled, and the catalyst is removed by filtration. The filtrate is concentrated and allowed to stand. The precipitated crystals are collected by filtration, dried and recrystallized from chloroform to give 1,2,3,5,6,7,8a - octahydro - 2 - oxo - imidazo[1,2 - a]pyridine - 3 - spiro - 4′ - piperidine as colorless crystals, m.p. 193°C. 2,3,5,6,7,8 - Hexahydro - 2 - oxo - imidazo[1,2 - a]pyridine - 3 - spiro - 4′ - piperidine is obtained from the mother liquor. Overall yield is 65%.

The compounds of formula (I) can form pharmaceutically acceptable acid addition salts with various inorganic and organic acids such as hydrochloric, hydrobromic, sulfuric, phosphoric, maleic, fumaric, oxalic, succinic and malonic acids.

The compounds of formula (I) and pharmaceutically acceptable acid addition salts thereof show psychotropic actions.

*Pharmacological Properties*

1. Test Compounds

A: 1′-[3-(10,11-dihydro-5H-dibenz[b,f]azepin-5-yl)propyl]-1,2,3,5,6,7,8,8a-octahydro-2-oxo-imidazo[1,2-a]pyridine-3-spiro-4′-piperidine dihydrochloride monohydrate

B: 1′-[3-(3-chloro-10,11-dihydro-5H-dibenz[b,f]azepin-5-yl)propyl]-1,2,3,5,6,7,8,8a-octahydro-2-oxo-imidazo[1,2-a]pyridine-3-spiro-4′-piperidine dihydrochloride monohydrate

C: 1′-[3-(10,11-dihydro-5H-dibenz[b,f]azepin-5-yl)propyl]-2,3,5,6,7,8-hexahydro-2-oxo-imidazo[1,2-a]pyridine-3-spiro-4′-piperidine dihydrochloride monohydrate

Chlorpromazine (Comparison).

2. Methods

(1) Effect on apomorphine-induced vomiting

The test was performed according to the method described by M. Nakanishi et al. in "Arzneimittel-Forschung", vol. 21, 391 ff (1971).

Groups of 5—10 beagle dogs of both sexes each were used. The test compound was administered orally 2 hours prior to the treatment with apomorphine hydrochloride (0.1 mg/kg, s.c.). The incidence of vomiting was checked for 2 hours, and the $ED_{50}$ (50% effective dose) was determined. The results are summarized in Table I.

(2) Cataleptogenic Activity

The test was performed according to the method described by W. Wirth et al. in "Archives Internationales de Pharmacodynamie et de Thérapie", vol, 115, 1 ff (1958).

Groups of 5 Wistar female rats each were used. The test compound was administered intraperitoneally, and the stages of catatonia induced with the test compound were observed in the same manner as described in the above-cited literature. The $ED_{50}$ was determined from total points of the maximum appearance of catatonia. The results are summarized in Table I.

3. Results

TABLE I

| Test Compound | Effect on Apomorphine-Induced Vomiting $ED_{50}$ (mg/kg, p.o.) | Cataleptogenic Activity $ED_{50}$ (mg/kg, i.p.) |
|---|---|---|
| A | 2.5 | > 50 |
| B | 0.5 | > 50 |
| C | 2.0 | > 50 |
| Chlorpromazine | 5.1 | 12 |

The compounds of the invention exhibit more potent than Chlorpromazine in effect on vomiting in dogs induced by apomorphine, one of desirable pharmacological properties, which permits such compounds to be employed as psychotropics. On the other hand, it is reported that the cataleptogenic activity in the animal corresponds to the side effects inducing extra-pyramidal symptoms in human being. In the experiment of catalepsy inducing activity, it is revealed that the compounds of the invention are by far weaker than Chlorpromazine.

In view of various tests including these mentioned above, the compounds of the invention represented by formula (I), in base or salt form, can be safely administered as psychotropics for the treatment of schizophrenia, depression, neurosis and the like in the form of a pharmaceutical preparation with a suitable and conventional pharmaceutically acceptable carrier, without adversely affecting the patients.

The pharmaceutical preparations can take any conventional form such as tablets, capsules, granules, powders or injectable solutions.

The following is an example of formulations when a compound of the invention is administred for pharmaceutical purposes:

Tablets (25 mg) are prepared from the following compositions:

| | |
|---|---|
| Compound I or its salt | 25 mg |
| Lactose | 60 mg |
| Cornstarch | 23 mg |
| Microcrystalline cellulose | 10 mg |
| Methyl cellulose | 1.5 mg |
| Magnesium stearate | 0.5 mg |
| | 120 mg |

The daily dose of compound (I) or a salt thereof for human adults usually ranges from about 10 mg to about 500 mg for oral administration, in single or multiple dose, but it may vary depending upon the age, body weight, and/or severity of the conditions to be treated as well as the response to the medication.

The present invention will be better understood from the following examples, but they are not to be construed as limiting the present invention.

Example 1

A mixture of 23 g of 5 - (3 - chloropropyl) - 10,11 - dihydro - 5H - dibenz[b,f]azepine, 15 g of 1,2,3,5,6,7,8,8a - octahydro - 2 - oxo - imidazo[1,2-a]pyridine - 3 - spiro - 4' - piperidine and 10 g of anhydrous potassium carbonate in 100 ml of ethanol is boiled under reflux for 48 hours. The ethanol is then distilled off under reduced pressure. The residue is added to 80 ml of toluene, and the insoluble matter is filtered off. The filtrate is washed with two 50 ml portions of water, and alcoholic hydrochloric acid is added. The precipitated crystals are collected by filtration and recrystallized from methanol to give 1' - [3 - (10,11 - dihydro - 5H - dibenz[b,f]azepin - 5 - yl)propyl]-1,2,3,5,6,7,8,8a - octahydro - 2 - oxo - imidazo[1,2 - a]pyridine - 3 - spiro - 4' - piperidine dihydrochloride monohydrate as white crystals, m.p. 258°C, 70% yield.

Example 2

A mixture of 5 g of 3 - chloro - 5 - (3 - methylsulfonyloxypropyl) - 10,11 - dihydro - 5H - dibenz[b,f]azepine, 4 g of 1,2,3,5,6,7,8,8a - octahydro - 2 - oxo - imidazo[1,2 - a]pyridine - 3 - spiro - 4' - piperidine, 4 g of potassium carbonate and 4 g of water in 40 ml of ethanol is stirred at 60°C for 4 hours. The upper layer is separated and concentrated, and the residue is dissolved in 100 ml of toluene. The toluene solution is washed with water, dried, concentrated to about 15 ml and allowed to stand. The precipitated crystals are collected by filtration and recrystallized from ethanol. The crystals thus obtained are dissolved in methanol, and methanolic hydrochloric acid is added to give 1' - [3 - (3 - chloro - 10,11 - dihydro - 5H - dibenz[b,f]azepin - 5 - yl) - propyl] - 1,2,3,5,6,7,8,8a - octahydro - 2 - oxo - imidazo[1,2 - a]pyridine - 3 - spiro - 4' - piperidine dihydrochloride monohydrate, m.p. 261°C. This product is dried at 120°C under reduced pressure for 4 hours to give the anhydrous product as white crystals, m.p. 271°C, 4.3 g.

Example 3

A mixture of 3.0 g of 3 - chloro - 5 - (3 - bromopropylidene) - 10,11 - dihydro - 5H - dibenz[a,d]cycloheptene and 3.0 g of 1,2,3,5,6,7,8,8a - octahydro - 2 - oxo - imidazo[1,2 - a]pyridine - 3 - spiro - 4' - piperidine in 20 ml of ethanol is boiled under reflux with stirring for 5 hours. The ethanol is then distilled off under reduced pressure, and the residue is dissolved in 30 ml of toluene. The toluene solution is washed with water and extracted with 30 ml of 5% hydrochloric acid. The aqueous layer is treated with activated charcoal and made alkaline. The separated oil is allowed to stand. The crystals thus obtained is dissolved in ethanol, and ethanolic hydrochloric acid is added to give 2.8 g of 1' - [3 - (3 - chloro - 10,11 - dihydro - 5H - dibenzo[a,d]cyclohepten - 5 - ylidene)propyl] - 1,2,3,5,6,7,8,8a - octahydro - 2 - oxo - imidazo[1,2 - a]pyridine - 3 - spiro - 4' - piperidine dihydrochloride as white crystals, m.p. 262°C.

Using the procedures set forth in the above examples, the following compounds are also produced:

(4) 1'-[3-(10,11-dihydro-5H-dibenz[b,f]azepin-5-yl)propyl]-2,3,5,6,7,8-hexahydro-2-oxo-imidazo[1,2-a]pyridine-3-spiro-4'-piperidine dihydrochloride monohydrate, m.p. 257°C

(5) 1'-[3-(5H-dibenz[b,f]azepin-5-yl)propyl]-1,2,3,5,6,7,8,8a-octahydro-2-oxo-imidazo[1,2-a]pyridine-3-spiro-4'-piperidine dihydrochloride monohydrate, m.p. 233°C

(6) 1'-[3-(5H-dibenz[b,f]azepin-5-yl)propyl]-2,3,5,6,7,8-hexahydro-2-oxo-imidazo[1,2-a]pyridine-3-spiro-4'-piperidine difumarate, m.p. 192—194°C

(7) 1'-[3-(3-chloro-10,11-dihydro-5H-dibenz[b,f]azepin-5-yl)propyl]-2,3,5,6,7,8-hexahydro-2-oxo-imidazo[1,2-a]pyridine-3-spiro-4'-piperidine dimaleate, m.p. 135—138°C

(8) 1'-[3-(3-bromo-10,11-dihydro-5H-dibenz[b,f]azepin-5-yl)propyl]-1,2,3,5,6,7,8,8a-octahydro-2-oxo-imidazo[1,2-a]pyridine-3-spiro-4'-piperidine dihydrochloride, m.p. 269—270°C

(9) 1'-[3-(3-chloro-5H-dibenz[b,f]azepin-5-yl)propyl]-1,2,3,5,6,7,8,8a-octahydro-2-oxo-imidazo[1,2-a]pyridine-3-spiro-4'-piperidine

(10) 1'-[3-(3-chloro-5H-dibenz[b,f]azepin-5-yl)propyl]-2,3,5,6,7,8-hexahydro-2-oxo-imidazo[1,2-a]pyridine-3-spiro-4'-piperidine

(11) 1'-[3-(3-methoxy-10,11-dihydro-5H-dibenz[b,f]azepin-5-yl)propyl]-1,2,3,5,6,7,8,8a-octahydro-2-oxo-imidazo[1,2-a]pyridine-3-spiro-4'-piperidine

The invention encompasses also the following compounds in their free form (non-salt form):

1'-[3-(10,11-dihydrol-5H-dibenz[b,f]azepin-5-yl)propyl]-1,2,3,5,6,7,8,8a-octahydro-2-oxo-imidazo[1,2-a]pyridine-3-spiro-4'-piperidine

1'-[3-(3-chloro-10,11-dihydro-5H-dibenz[b,f]azepin-5-yl)propyl]-1,2,3,5,6,7,8,8a-octahydro-2-oxo-imidazo[1,2-a]pyridine-3-spiro-4'-piperidine

1'-[3-(10,11-dihydro-5H-dibenz[b,f]azepin-5-yl)propyl]-2,3,5,6,7,8-hexahydro-2-oxo-imidazo[1,2-a]pyridine-3-spiro-4'-piperidine

1'-[3-(5H-dibenz[b,f]azepin-5-yl)propyl]-1,2,3,5,6,7,8,8a-octahydro-2-oxo-imidazo[1,2-a]pyridine-3-spiro-4'-piperidine

1'-[3-(5H-dibenz[b,f]azepin-5-yl)propyl]-2,3,5,6,7,8-hexahydro-2-oxo-imidazo[1,2-a]pyridine-3-spiro-4'-piperidine

1'-[3-(3-chloro-10,11-dihydro-5H-dibenz[b,f]azepin-5-yl)propyl]-2,3,5,6,7,8-hexahydro-2-oxo-imidazo[1,2-a]pyridine-3-spiro-4'-piperidine

1'-[3-(3-bromo-10,11-dihydro-5H-dibenz[b,f]azepin-5-yl)propyl]-1,2,3,5,6,7,8,8a-octahydro-2-oxo-imidazo[1,2-a]pyridine-3-spiro-4'-piperidine, and

1′-[3-(3-chloro-10,11-dihydro-5H-dibenzo[a,d]cyclohepten-5-ylidene)propyl]-1,2,3,5,6,7,8,8a-octahydro-2-oxo-imidazo[1,2-a]pyridine-3-spiro-4′-piperidine.

**Claims**

1. An imidazopyridine-spiro-piperidine compound of the formula:

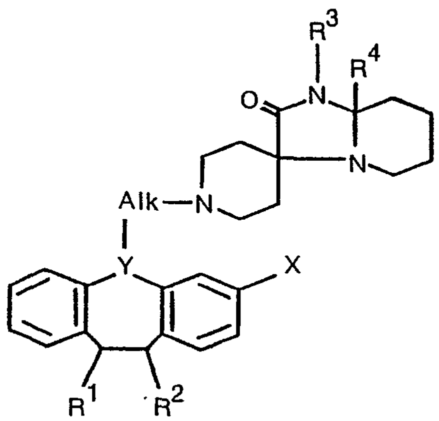

(I)

or a pharmaceutically acceptable acid addition salt thereof, wherein X represents hydrogen, halogen or lower alkoxy selected from methoxy, ethoxy, propoxy or butoxy, Y represents

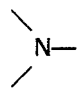

or

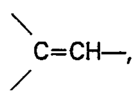

$R^1$ and $R^2$ both represent hydrogen or combinedly form a single bond, Alk represents lower alkylene selected from ethylene, propylene or trimethylene, and $R^3$ and $R^4$ both represent hydrogen or combinedly form a single bond.

2. The compound of Claim 1: 1′ - [3 - (10,11 - dihydro - 5H - dibenz[b,f]azepin - 5 - yl)propyl] - 1,2,3,5,6,7,8,8a - octahydro - 2 - oxo - imidazo[1,2 - a]pyridine - 3 - spiro - 4′ - piperidine and its pharmaceutically acceptable acid addition salts.

3. The compound of Claim 1: 1′ - [3 - (3 - chloro - 10,11 - dihydro - 5H - dibenz[b,f]azepin - 5 - yl)propyl] - 1,2,3,5,6,7,8,8a - octahydro - 2 - oxo - imidazo[1,2 - a]pyridine - 3 - spiro - 4′ - piperidine and its pharmaceutically acceptable acid addition salts.

4. The compound of Claim 1: 1′ - [3 - (10,11 - dihydro - 5H - dibenz[b,f]azepin - 5 - yl)propyl] - 2,3,5,6,7,8 - hexahydro - 2 - oxo - imidazo[1,2 - a]pyridine - 3 - spiro - 4′ - piperidine and its pharmaceutically acceptable acid addition salts.

5. The compound of Claim 1: 1′ - [3 - (5H - dibenz[b,f]azepin - 5 - yl)propyl] - 1,2,3,5,6,7,8,8a - octahydro - 2 - oxo - imidazo[1,2 - a]pyridine - 3 - spiro - 4′ - piperidine and its pharmaceutically acceptable acid addition salts.

6. The compound of Claim 1: 1′ - [3 - (5H - dibenz[b,f]azepin - 5 - yl)propyl] - 2,3,5,6,7,8 - hexahydro - 2 - oxo - imidazo[1,2 - a]pyridine - 3 - spiro - 4′ - piperidine and its pharmaceutically acceptable acid addition salts.

7. The compound of Claim 1: 1′ - [3 - (3 - chloro - 10,11 - dihydro - 5H - dibenz[b,f]azepin - 5 - yl)propyl] - 2,3,5,6,7,8 - hexahydro - 2 - oxo - imidazo[1,2 - a]pyridine - 3 - spiro - 4′ - piperidine and its pharmaceutically acceptable acid addition salts.

8. The compound of Claim 1: 1′ - [3 - (3 - bromo - 10,11 - dihydro - 5H - dibenz[b,f]azepin - 5 - yl)propyl] - 1,2,3,5,6,7,8,8a - octahydro - 2 - oxo - imidazo[1,2 - a]pyridine - 3 - spiro - 4′ - piperidine and its pharmaceutically acceptable acid addition salts.

9. The compound of Claim 1: 1′ - [3 - (3 - chloro - 10,11 - dihydro - 5H - dibenzo[a,d]cyclohepten - 5 - ylidene)propyl] - 1,2,3,5,6,7,8,8a - octahydro - 2 - oxo - imidazo[1,2 - a]pyridine - 3 - spiro - 4′ - piperidine and its pharmaceutically acceptable acid addition salts.

10. A pharmaceutical composition comprising a compound of claims 1 to 9 in combination with a pharmaceutically acceptable inert carrier, said compound being present in a therapeutically effective amount.

**Patentansprüche**

1. Imidazopyridin-spiro-piperidin-Verbindung der Formel

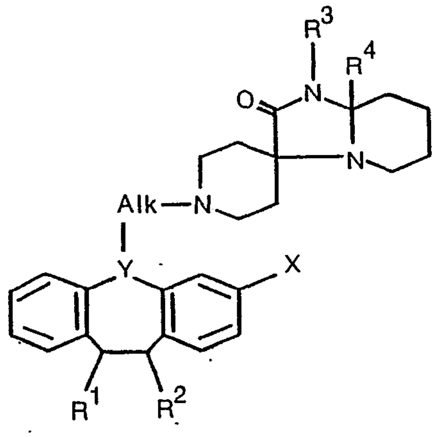

worin

X Wasserstoff, Halogen oder Niederalkoxy ausgewählt aus Methoxy, Ethoxy, Propoxy oder Butoxy bezeichnet,

Y

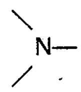

oder

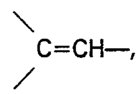

bezeichnet,

$R^1$ und $R^2$ beide Wasserstoff bezeichnen oder miteinander kombiniert eine Einfachbindung bilden,

Alk Niederalkylen ausgewählt aus Ethylen, Propylen oder Trimethylen bezeichnet und

$R^3$ und $R^4$ beide Wasserstoff bezeichnen oder miteinander kombiniert eine Einfachbindung bilden, oder ein pharmazeutisch annehmbares Säureadditionssalz derselben.

2. Verbindung nach Anspruch 1: 1' - [3 - (10,11 - Dihydro - 5H - dibenz[b,f]azepin - 5 - yl)propyl] - 1,2,3,5,6,7,8,8a - octahydro - 2 - oxo - imidazo[1,2a]pyridin - 3 - spiro - 4' - piperidin und dessen pharmazeutisch annehmbare Säureadditionssalze.

3. Verbindung nach Anspruch 1: 1' - [3 - (3 - Chloro - 10,11 - dihydro - 5H - dibenz[b,f]azepin - 5 - yl)propyl] - 1,2,3,5,6,7,8,8a - octahydro - 2 - oxo - imidazo[1,2a]pyridin - 3 - spiro - 4' - piperidin und dessen pharmazeutisch annehmbare Säureadditionssalze.

4. Verbindung nach Anspruch 1: 1' - [3 - (10,11 - Dihydro - 5H - dibenz[b,f]azepin - 5 - yl)propyl] - 2,3,5,6,7,8 - hexahydro - 2 - oxo - imidazo[1,2a]pyridin - 3 - spiro - 4' - piperidin und dessen pharmazeutisch annehmbare Säureadditionssalze.

5. Verbindung nach Anspruch 1: 1' - [3 - (5H - Dibenz[b,f]azepin - 5 - yl)propyl] - 1,2,3,5,6,7,8,8a - octahydro - 2 - oxo - imidazo[1,2a]pyridin - 3 - spiro - 4' - piperidin und dessen pharmazeutisch annehmbare Säureadditionssalze.

6. Verbindung nach Anspruch 1: 1' - [3 - (5H - Dibenz[b,f]azepin - 5 - yl)propyl] - 2,3,5,6,7,8 - hexahydro - 2 - oxo - imidazo[1,2a]pyridin - 3 - spiro - 4' - piperidin und dessen pharmazeutisch annehmbare Säureadditionssalze.

7. Verbindung nach Anspruch 1: 1' - [3 - (3 - Chloro - 10,11 - dihydro - 5H - dibenz[b,f]azepin - 5 - yl)propyl] - 2,3,5,6,7,8 - hexahydro - 2 - oxo - imidazo[1,2a]pyridin - 3 - spiro - 4' - piperidin und dessen pharmazeutisch annehmbare Säureadditionssalze.

8. Verbindung nach Anspruch 1: 1' - [3 - (3 - Bromo - 10,11 - dihydro - 5H - dibenz[b,f]azepin - 5 -

yl)propyl] - 1,2,3,5,6,7,8,8a - octahydro - 2 - oxo - imidazo[1,2a]pyridin - 3 - spiro - 4′ - piperidin und dessen pharmazeutisch annehmbare Säureadditionssalze.

9. Verbindung nach Anspruch 1: 1′ - [3 - (3 - Chloro - 10,11 - dihydro - 5H - dibenzo[a,d]cyclohepten - 5 - yliden)propyl] - 1,2,3,5,6,7,8,8a - octahydro - 2 - oxo - imidazo[1,2a]pyridin - 3 - spiro - 4′ - piperidin und dessen pharmazeutisch annehmbare Säureadditionssalze.

10. Pharmazeutische Zusammensetzung, enthaltend eine Verbindung nach den Ansprüchen 1 bis 9 in Kombination mit einem pharmazeutisch annehmbaren inerten Träger, wobei die Verbindung in therapeutisch wirksamer Menge vorliegt.

**Revendications**

1. Dérivé d'imidazopyridine-spiro-pipéridine de formule:

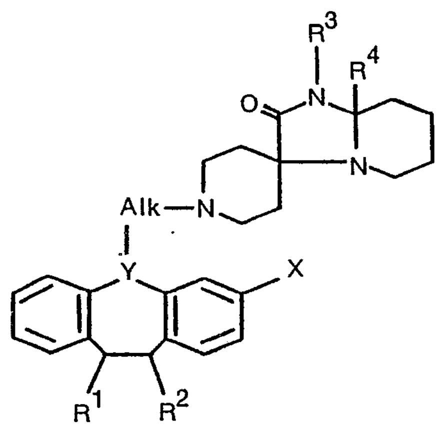

ou un de ses sels d'addition avec des acides pharmaceutiquement acceptables, formule dans laquelle X représente un hydrogène, un halogène, ou un alcoxy inférieur choisi parmi les méthoxy, éthoxy, propoxy ou butoxy, Y représente

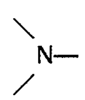

ou

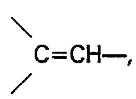

$R^1$ et $R^2$ représentent tous les deux un hydrogène ou forment, en combinaison, une double liaison, Alk représente un alkylène inférieur choisi parmi les éthylène, propylène ou triméthylène et $R^3$ et $R^4$ représentent tous les deux un hydrogène ou forment, en combinaison, une double liaison.

2. Composé selon la revendication 1, la 1′ - [3 - (10,11 - dihydro - 5H - dibenz[b,f]azépin - 5 - yl)propyl] - 1,2,3,5,6,7,8,8a - octahydro - 2 - oxo - imidazo[1,2 - a]pyridine - 3 - spiro - 4′ - pipéridine et ses sels d'addition avec des acides pharmaceutiquement acceptables.

3. Composé selon la revendication 1, la 1′ - [3 - (3 - chloro - 10,11 - dihydro - 5H - dibenz - [b,f]azépin - 5 - yl)propyl] - 1,2,3,5,6,7,8,8a - octahydro - 2 - oxo - imidazo[1,2 - a]pyridine - 3 - spiro - 4′ - pipéridine et ses sels d'addition avec des acides pharmaceutiquement acceptables.

4. Composé selon la revendication 1, la 1′ - [3 - (10,11 - dihydro - 5H - dibenz[b,f]azépin - 5 - yl)propyl] - 2,3,5,6,7,8 - hexahydro - 2 - oxo - imidazo[1,2 - a]pyridine - 3 - spiro - 4′ - pipéridine et ses sels d'addition avec des acides pharmaceutiquement acceptables.

5. Composé selon la revendication 1, la 1′ - [3 - (5H - dibenz[b,f]azépin - 5 - yl)propyl] - 1,2,3,5,6,7,8,8a - octahydro - 2 - oxo - imidazo[1,2 - a]pyridine - 3 - spiro - 4′ - pipéridine et ses sels d'addition avec des acides pharmaceutiquement acceptables.

6. Composé selon la revendication 1, la 1′ - [3 - (5H - dibenz[b,f]azépin - 5 - yl)propyl] - 2,3,5,6,7,8 - hexahydro - 2 - oxo - imidazo[1,2 - a]pyridine - 3 - spiro - 4′ - pipéridine et ses sels d'addition avec des acides pharmaceutiquement acceptables.

7. Composé selon la revendication 1, la 1′ - [3 - (3 - chloro - 10,11 - dihydro - 5H -

dibenz[b,f]azépin - 5 - yl)propyl] - 2,3,5,6,7,8 - hexahydro - 2 - oxo - imidazo[1,2 - a]pyridine - 3 - spiro - 4' - pipéridine et ses sels d'addition avec des acides pharmaceutiquement acceptables.

8. Composé selon la revendication 1, la 1' - [3 - (3 - bromo - 10,11 - dihydro - 5H - dibenz[b,f]azépin - 5 - yl)propyl] - 1,2,3,5,6,7,8,8a - octahydro - 2 - oxo - imidazo[1,2 - a]pyridine - 3 - spiro - 4' - pipéridine et ses sels d'addition avec des acides pharmaceutiquement acceptables.

9. Composé selon la revendication 1, la 1' - [3 - (3 - chloro - 10,11 - dihydro - 5H - dibenzo[a,d]cyclohepten - 5 - ylidène)propyl] - 1,2,3,5,6,7,8,8a - octahydro - 2 - oxo - imidazo[1,2 - a]pyridine - 3 - spiro - 4' - pipéridine et ses sels d'addition avec des acides pharmaceutiquement acceptables.

10. Composition pharmaceutique, caractérisée en ce qu'elle contient un composé selon les revendications 1 à 9, en association avec un véhicule inerte pharmaceutiquement acceptable, ledit composé étant présent à une dose thérapeutiquement efficace.